# EUROPEAN PATENT APPLICATION

(11) **EP 3 574 892 A1**
(43) Date of publication of application: **04.12.2019**
(21) Application number: 17913270.9
(22) Date of filing: 14.06.2017
(51) Int. Cl.: A61K 8/64, A61K 8/23, A61Q 19/08

(54) **COSMETIC COMPOSITION FOR WRINKLE REDUCTION OR ANTI-INFLAMMATION, CONTAINING SUBSTANCE P**

(71) Applicant: Bio Solution Co., Ltd., Seoul 01811 (KR)
(72) Inventor: KIM, Da Jung, Seoul 01811 (KR); LEE, Jung Sun, Seoul 01811 (KR); JANG, Song Sun, Seoul 01811 (KR)
(74) Representative: Goodall, Scott
(86) International application number: PCT/KR2017/006218
(87) International publication number: WO 2018/230751

(57) **Abstract**

Provided are a composition for improving skin wrinkles or inflammation, the composition including substance P as an active ingredient, more particularly, novel use of a composition including substance P which promotes collagen synthesis, inhibits collagenase production, and performs anti-inflammatory responses to improve skin wrinkles or inflammation, and a cosmetic composition for improving skin wrinkles or inflammation including the composition.

The composition of the present invention, which includes substance P, exhibits higher effects of improving skin wrinkle or inflammation than substance P alone, due to a synergistic effect of an antioxidant, a surfactant, and a thickener which are added for stability of substance P, together with basic characteristics of substance P which is known to have effects of improving skin wrinkles or inflammation. Accordingly, the composition of the present invention may be usefully applied to a cosmetic composition for improving skin wrinkles or inflammation.

## Description

### [Technical Field]

The present invention relates to a cosmetic composition for improving skin wrinkles or inflammation, the cosmetic composition including substance P as an active ingredient. More particularly, the present invention relates to a cosmetic composition having anti-wrinkle or anti-inflammatory effects by promoting collagen synthesis, inhibiting collagenase (MMP-1) synthesis, and performing anti-inflammatory responses.

### [Background Art]

The cosmetics industry tends to grow steadily. In a variety of cosmetics-related industries, skin aging-related cosmetic products are considered to be the fastest-growing sector. Skin aging is caused by various factors. The factors that directly affect skin aging may be largely divided into wrinkle formation due to collagen reduction in the skin and inflammatory reaction due to oxidative stress.

Collagen which is a major component of the extracellular matrix is a major matrix protein produced by skin fibroblasts. Collagen is known to have functions such as skin firmness, binding force of connective tissues and skin tissues, support of cell adhesion, induction of cell division and differentiation, and thus it plays a major role in maintaining skin elasticity by increasing water retention in the dermis. Such collagen is reduced by aging and photoaging by UV radiation, and the collagen reduction is promoted by the activity of collagenase that degrades collagen, which is involved in the formation of skin wrinkles.

Generally, products obtained by blending collagen with a skin external composition such as cosmetics or ointments are brought into the market to take an advantage of the wrinkle-improving effect of collagen. However, these products are to apply collagen itself to the surface of skin. Since collagen is a large molecule, it is hardly absorbed through the skin, and thus intrinsic anti-wrinkle effect cannot be expected. To solve this problem, collagen synthesis-promoting materials have attracted much attention. Examples of generally known collagen synthesis-promoting materials include retinoid, adenosine, a chlorella extract (JP9-40523, JP10-36283, promoting proliferation of fibroblasts), vitamin C, a transforming growth factor (TGF), protein originating from animal placenta (JP8-231370), betulinic acid (JP8-208424), *etc.*

Among them, the most widely known retinol promotes collagen synthesis, but it causes skin irritation, redness, *etc*., when used as a cosmetic material. Its application is also limited, because it is unstable to light. The effect of the chlorella extract is not noticeable, and thus it is difficult to expect skin wrinkle-improving effects. As described, use of these substances is limited due to safety problems such as irritation and redness when applied to the skin, or their effects are not noticeable. Thus, there is a problem in that wrinkle-improving effects cannot be practically expected. Accordingly, there is an urgent need to develop a novel anti-wrinkle composition which is safer to the living body and has higher a wrinkle-improving effect than the general compositions for improving wrinkles.

Meanwhile, inflammation is a part of the immune responses against diseases or wounds of the human body. During the inflammatory responses, various free radicals are produced along with various inflammatory factors. Free radicals are normally involved in maintaining the homeostasis of cells and affect cell differentiation, growth, survival, and aging. Of free radicals, reactive oxygen species (ROS) is constantly produced through oxidation and reduction of oxygen by respiration and immune responses in mitochondria within the cell. When an imbalance of free radical generation and scavenging occurs, oxidative stress is developed to activate inflammatory factors, leading to cell damage and skin aging.

Inflammatory responses are characterized by an increase of inflammatory cytokines (TNF-alpha, IL-6, *etc*.) and a decrease of anti-inflammatory cytokines (IL-4, IL-10, *etc*.)*.* Further, a strong inflammatory mediator, nitric oxide (NO), is produced by NO synthases, and is generated in many different kinds of cells by photoaging stress or cytokines.

Anti-inflammatory materials capable of improving skin aging include nonsteroidal flufenamic acid benzydamine, and steroidal prednisolone and dexamethasone. However, these materials also have disadvantages in that they have low safety to the skin and their anti-inflammatory effects are not noticeable.

Meanwhile, substance P is a neurotransmitter composed of 11 amino acids, and is known to be expressed in various kinds of cells and granulation tissues. Several studies have reported that substance P remarkably enhances collagen remodeling in primary human hamstering tenocytes, and inhibits transepidermal water loss in the artificial skin tissue to prevent water loss from stratum corneum, thereby maintaining water retention in the stratum corneum. Further, substance P is also known to contribute to early termination of inflammatory responses by decreasing inflammation-related leukocytes, neutrophils, and hematopoietic stem cells in the blood and by increasing anti-inflammatory cytokines, regulatory T lymphocytes, and anti-inflammatory macrophages (Korean Patent No. 10-1292451B1), suggesting that substance P may be suitably used as a composition for improving wrinkles and inflammatory responses. However, reduction of the anti-wrinkle and anti-inflammatory effects due to its instability is required to be solved, in order to use substance P in anti-wrinkle and anti-inflammatory cosmetics.

### [Disclosure]

### [Technical Problem]

Under this background, the present inventors have made many efforts to increase stability of substance P, and as a result, they found that a composition including substance P, an antioxidant, a surfactant, and a thickener as a composition previously developed by the present inventors is stable in a skin fibroblast growth medium, and at the same time, has excellent anti-wrinkle or anti-inflammatory effect, as compared with substance P alone, thereby completing the present invention.

### [Technical Solution]

An object of the present invention is to provide a cosmetic composition for improving skin wrinkles or inflammation, the cosmetic composition including substance P, a method of improving skin wrinkles or inflammation, the method including the step of applying the composition to the skin of a subject, and use of the composition.

### [Advantageous Effects]

The cosmetic composition of the present invention, which includes substance P, an antioxidant, a surfactant, and a thickener, may be widely used as a cosmetic composition for improving skin wrinkles or inflammation.

### [Description of Drawings]

FIG. 1 is a graph showing stability of substance P according to contents of an antioxidant and a surfactant;
FIG. 2 is a graph showing stability of a composition including substance P in a skin fibroblast culture medium;
FIG. 3 is a graph showing a collagen synthesis effect of a composition including substance P;
FIG. 4 is a graph showing collagenase inhibition of a composition including substance P;
FIG. 5 is a graph showing an inflammatory cytokine IL-6-reducing effect of a composition including substance P (a) and substance P alone (b); and
FIG. 6 is a graph showing a nitric oxide (NO)-reducing effect of a composition including substance P (a) and substance P (b) alone.

### [Best Mode]

To achieve the above objects, an aspect of the present invention provides a cosmetic composition for improving skin wrinkles or inflammation, the cosmetic composition including substance P, an antioxidant, a surfactant, and a thickener.

The present invention relates to a novel cosmetic composition for improving anti-wrinkle or anti-inflammatory effects which are reduced due to instability of substance P. The present inventors have developed components and contents of the composition which are optimized to improve anti-wrinkle or anti-inflammatory effects when substance P is applied.

Specifically, the present invention provides a cosmetic composition for improving skin wrinkles or inflammation, the cosmetic composition including substance P, sodium thiosulfate, polysorbate 80, and hydroxyethyl cellulose.

The present inventors found that the composition including substance P is stable in a skin fibroblast growth medium, promotes collagen synthesis, inhibits collagenase production, and performs anti-inflammatory responses to exhibit anti-wrinkle or anti-inflammatory effects, thereby completing the present invention.

In the composition of the present invention, the substance P refers to a neuropeptide composed of amino acids of "Arg-Pro-Lys-Pro-Gln-Gln-Phe-Phe-Gly-Leu-Met-NH₂" of SEQ ID NO: 1. The substance P is widely distributed in the central nervous systems such as the brain and spinal cord and peripheral organs such as intestinal tract, and functions to transmit pain sensation in the primary sensory neuron.

A concentration of the substance P in the cosmetic composition of the present invention may be 1 µg/mL to 10 µg/mL, and specifically, 5 µg/mL to 10 µg/mL.

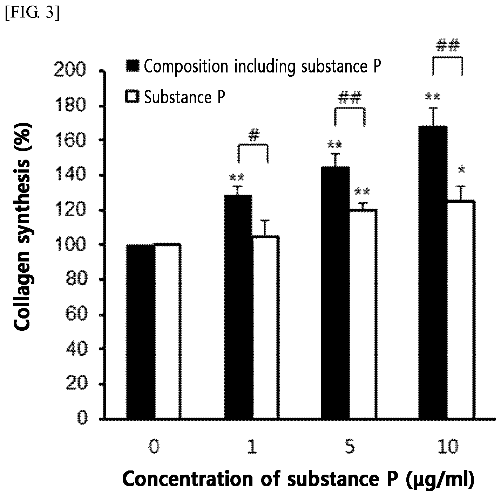

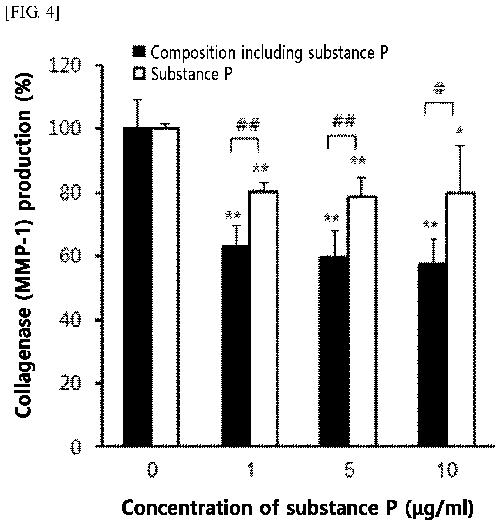

In one embodiment of the present invention, it was demonstrated that a composition including substance P at a concentration of 1 µg/mL to 10 µg/mL had excellent effects of synthesizing collagen and inhibiting collagenase, and the most excellent effects were observed in the range of 5 µg/mL to 10 µg/mL (FIGS. 3 and 4).

Therefore, it was confirmed that when the cosmetic composition of the present invention includes substance P at a concentration of 1 µg/mL to 10 µg/mL, the cosmetic composition of the present invention may exhibit an excellent anti-wrinkle effect.

In the composition of the present invention, the antioxidant refers to a substance that is added for the purpose of terminating chain reactions of oxidation by acting on free radicals or peroxide generated during oxidation of active ingredients by oxygen in the air, and preventing progress of oxidation and deterioration of active ingredients.

In the present invention, the antioxidant may prevent deterioration of anti-wrinkle or anti-inflammatory effects of the cosmetic composition including substance P.

As the antioxidant, an antioxidant commonly used in the art may be used without limitation. Specifically, β-mercaptoethanol (β-ME), glutathione (GSH), ascorbic acid, vitamin E, beta-carotene, lycopene, coenzyme Q-10, selenium, chromium, magnesium, taurine, hypotaurine, trehalose, *etc.* may be used, but it is not limited thereto. In the present invention, the antioxidant may be specifically sodium thiosulfate.

A content of the antioxidant is not particularly limited, as long as it is able to prevent deterioration of the anti-wrinkle or anti-inflammatory effects of the cosmetic composition, but the content may be 0.01% by weight to 1% by weight, and specifically, 0.1 % by weight to 1% by weight with respect to the total weight of the composition of the present invention.

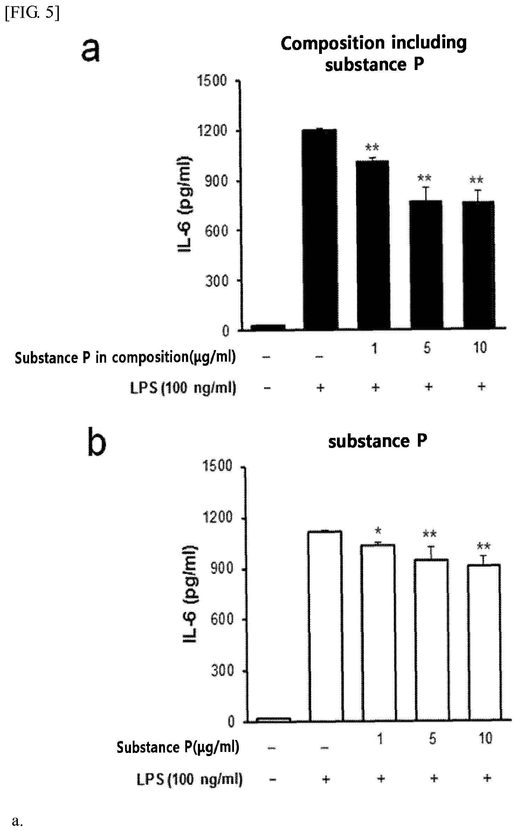

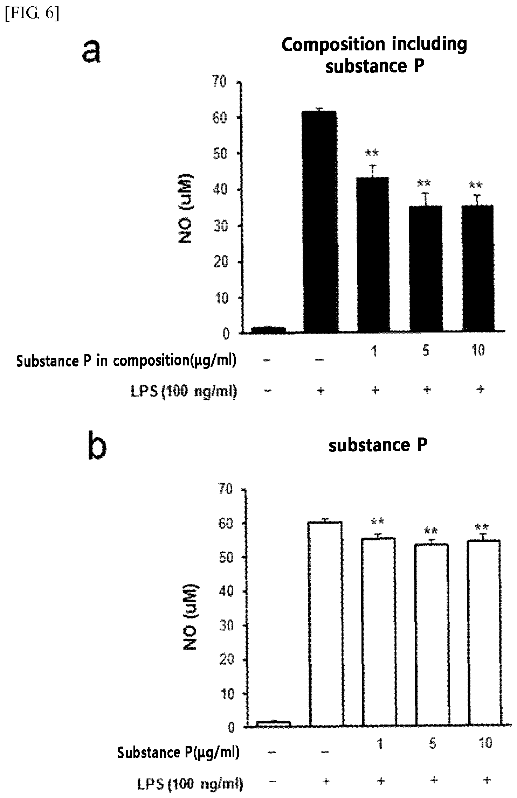

In one embodiment of the present invention, it was confirmed that a cosmetic composition including sodium thiosulfate as the antioxidant exhibited effects of promoting collagen synthesis and inhibiting collagenase (MMP-1) (FIGS. 3 and 4) and effects of reducing an inflammatory cytokine IL-6 and nitric oxide (NO) (FIGS. 5 and 6), which are superior to those of substance P alone.

Accordingly, when the cosmetic composition of the present invention includes sodium thiosulfate as the antioxidant, it may exhibit excellent anti-wrinkle or anti-inflammatory effects.

In the composition of the present invention, the surfactant refers to a substance that helps maintain a uniform liquid composition using hydrophobic oil components.

The surfactant may be a surfactant commonly used in the preparation of cosmetic compositions, such as anionic, cationic, non-ionic, or amphiphilic surfactants. Specifically, in the present invention, the surfactant may be polysorbate 80.

A content of the surfactant may be 0.001% by weight to 0.1% by weight, and specifically 0.006% by weight to 0.1% by weight with respect to the total weight of the composition of the present invention. In one embodiment of the present invention, it was confirmed that a cosmetic composition including polysorbate 80 as the surfactant exhibited effects of promoting collagen synthesis and inhibiting collagenase (MMP-1) (FIGS. 3 and 4) and effects of reducing an inflammatory cytokine IL-6 and nitric oxide (NO) (FIGS. 5 and 6), which are superior to those of substance P alone.

Accordingly, when the cosmetic composition of the present invention includes polysorbate 80 as the surfactant, it may exhibit excellent anti-wrinkle or anti-inflammatory effects.

In the composition of the present invention, the thickener refers to an additive that is added to provide viscosity, and is also called a thickening agent or thickening stabilizer. The thickener of the present invention may be specifically hydroxyethyl cellulose.

A content of the thickener may be 1% by weight to 5% by weight with respect to the total weight of the composition of the present invention. If the content of the thickener is 1% by weight or less with respect to the total weight of the composition, there is a problem in the stability of the cosmetic composition. If the content of the thickener is 5% by weight or more with respect to the total weight of the composition, viscosity of the cosmetic composition is excessively increased and thus the composition becomes unsuitable for application.

In one embodiment of the present invention, it was confirmed that a cosmetic composition including hydroxyethyl cellulose as the thickener exhibited effects of promoting collagen synthesis and inhibiting collagenase (MMP-1) (FIGS. 3 and 4) and effects of reducing an inflammatory cytokine IL-6 and nitric oxide (NO) (FIGS. 5 and 6), which are superior to those of substance P alone.

Accordingly, when the cosmetic composition of the present invention includes hydroxyethyl cellulose as the thickener, it may exhibit excellent anti-wrinkle or anti-inflammatory effects.

Specifically, the present invention provides a cosmetic composition for improving skin wrinkles or inflammation, the cosmetic composition including substance P, sodium thiosulfate as the antioxidant, polysorbate 80 as the surfactant, and hydroxyethyl cellulose as the thickener.

Specifically, the present invention provides a cosmetic composition for improving skin wrinkles or inflammation, the cosmetic composition including substance P, 0.01% by weight to 1% by weight of the antioxidant, 0.001% to 0.1% by weight of the surfactant, and 1% to 5% by weight of the thickener.

More specifically, the present invention provides a cosmetic composition for improving skin wrinkles or inflammation, the cosmetic composition including 0.1% by weight to 1% by weight of the antioxidant and 0.006% to 0.1% by weight of the surfactant.

Further, specifically, the present invention provides a cosmetic composition for improving skin wrinkles or inflammation, the cosmetic composition including substance P, 0.01% by weight to 1% by weight of sodium thiosulfate as the antioxidant, 0.001% by weight to 0.1% by weight of polysorbate 80 as the surfactant, and 1% by weight to 5% by weight of hydroxyethyl cellulose as the thickener. More specifically, the present invention provides a cosmetic composition for improving skin wrinkles or inflammation, the cosmetic composition including substance P, 0.1% by weight to 1% by weight of sodium thiosulfate as the antioxidant and 0.006% by weight to 0.1% by weight of polysorbate 80 as the surfactant.

In one embodiment of the present invention, it was demonstrated that the cosmetic composition including substance P, the antioxidant, the surfactant, and the thickener exhibited effects of promoting collagen synthesis and inhibiting collagenase (MMP-1) (FIGS. 3 and 4), which are superior to those of substance P alone, and therefore, it was confirmed that the cosmetic composition has superior anti-wrinkle effects, as compared with substance P alone.

Further, in one embodiment of the present invention, it was demonstrated that the cosmetic composition including substance P, the antioxidant, the surfactant, and the thickener exhibited effects of reducing an inflammatory cytokine IL-6 (FIG. 5) and nitric oxide (NO) (FIG. 6), which are superior to those of substance P alone, and therefore, it was confirmed that the cosmetic composition has superior anti-inflammation effects, as compared with substance P alone.

Accordingly, the cosmetic composition of the present invention may be usefully applied to cosmetics for improving skin wrinkles or inflammation.

As used herein, the term "cosmetic composition" may be prepared in the form of a general emulsion formulation and a solubilized formulation. The emulsion formulation may include a nutrition lotion, a cream, an essence, *etc*., and the solubilized formulation may include a soft lotion, *etc.* The cosmetic composition may be prepared as a formulation selected from the group consisting of a solution, a suspension, an emulsion, a paste, a gel, a cream, a lotion, a powder, a soap, a surfactant-containing cleansing, an oil, a powdered foundation, an emulsified foundation, a wax foundation, and a spray, but is not limited thereto. Specifically, the cosmetic composition may be prepared in the form of a hypoallergenic cosmetic skin protector, a soft lotion, a nutrition lotion, a nutrition cream, a massage cream, an essence, an eye cream, a serum, a cleansing cream, a cleansing foam, a cleansing water, a pack, a cream, an essence, a spray, or a powder.

Further, the cosmetic composition may further include one or more cosmetically acceptable carriers which are commonly blended in a general skin cosmetic material. A common ingredient, *e.g*., oil, water, a surfactant, a moisturizer, lower alcohol, a thickener, a chelating agent, a pigment, a preservative, a perfume, *etc.* may be appropriately blended, but is not limited thereto.

The cosmetically acceptable carrier included in the cosmetic composition may vary depending on the formulation.

When the formulation of the cosmetic composition is an ointment, a paste, a cream, or a gel, animal oil, vegetable oil, wax, paraffin, starch, tragacanth, a cellulose derivative, polyethylene glycol, silicone, bentonites, silica, talc, zinc oxide, or a mixture thereof may be used as the carrier component.

When the formulation of the cosmetic composition is a powder or spray, lactose, talc, silica, aluminum hydroxide, calcium silicate, polyamide powder, or a mixture thereof may be used as the carrier component. In particular, when the formulation of the cosmetic composition is a spray, a propellant such as chlorofluorohydrocarbon, propane/butane, or dimethyl ether may be additionally included.

When the formulation of the cosmetic composition is a solution or an emulsion, a solvent, a solubilizer, or an emulsifying agent may be used as the carrier component. For example, water, ethanol, isopropanol, ethyl carbonate, ethyl acetate, benzyl alcohol, benzyl benzoate, propylene glycol, or 1,3-butyl glycol oil may be used. In particular, cottonseed oil, peanut oil, corn oil, olive oil, castor oil and sesame oil, glycerol aliphatic esters, fatty acid esters of polyethylene glycol or sorbitan may be used.

When the formulation of the cosmetic composition is a suspension, a liquid diluent such as water, ethanol, or propylene glycol; a suspension such as ethoxylated isostearyl alcohol, polyoxyethylene sorbitol ester, and polyoxyethylene sorbitan ester; microcrystalline cellulose, aluminum meta-hydroxide, bentonite, agar, or tragacanth may be used as the carrier component.

When the formulation of the cosmetic composition is soap, alkali metal salts of fatty acids, salts of fatty acid hemiesters, fatty acid protein hydrolysate, isethionate, lanolin derivatives, aliphatic alcohol, vegetable oil, glycerol, sugars may be used as the carrier component.

As used herein, the term "wrinkle improvement" means that occurrence of wrinkles in the skin is inhibited or impeded, or already formed wrinkles are alleviated.

As used herein, the term "inflammation improvement or anti-inflammation" means an inflammation-inhibiting action. Inflammation is one of defense mechanisms in the living body against external stimuli, and specifically, is one of innate immune responses, whereby external stimuli, particularly, specific patterns on the surface of pathogens are recognized. Inflammatory cells in the living body recognize specific patterns on the surfaces as a non-self to attack pathogens. At this time, when pathogens break through physical barriers and invade the living body, inflammatory responses occur. At the early stage of inflammatory responses, white blood cells responsible for the immune responses express inflammatory cytokines. Thus, the intracellular expression level of the cytokines is an indicator of inflammatory activation. Further, a strong inflammatory mediator (nitric oxide (NO)) may also be an additional indicator of inflammatory responses, because it is generated in many kinds of cells by cytokines.

Another aspect of the present invention provides a method of improving skin wrinkles or inflammation, the method including the step of administering, to a skin wrinkle- or inflammation-induced subject, the cosmetic composition for improving skin wrinkles or inflammation, the cosmetic composition including substance P, the antioxidant, the surfactant, and the thickener.

As used herein, the term "subject" refers to any animal including human susceptible to development of skin wrinkles or inflammation. The animal may be not only a human but also a mammal including cow, horse, sheep, pig, goat, camel, antelope, dog, and cat in need of treatment of similar symptoms, but is not limited thereto.

As used herein, the term "substance P", "antioxidant", "surfactant", and "thickener" are the same as described above.

The cosmetic composition of the present invention may be administered daily or intermittently, and may be used alone or in combination with other compositions to improve skin wrinkles or inflammation. Considering all the above factors, it is important to administer a minimum amount that may achieve a maximum effect without adverse effects, which may be readily determined by those skilled in the art.

Still another aspect of the present invention provides a method of improving skin wrinkles or inflammation, the method including the step of applying the cosmetic composition to the skin of a subject.

The cosmetic composition, and the skin wrinkle and inflammation improvement are the same as described above.

In one embodiment of the present invention, it was confirmed that the cosmetic composition including substance P, the antioxidant, the surfactant, and the thickener of the present invention exhibited effects of promoting collagen synthesis (FIG. 3) and inhibiting collagenase (MMP-1) (FIG. 4) and effects of reducing an inflammatory cytokine IL-6 (FIG. 5) and reducing nitric oxide (NO) (FIG. 6), which are superior to those of substance P alone. Accordingly, it was confirmed to provide the method of improving skin wrinkles or inflammation, the method including the step of applying the cosmetic composition including substance P to the skin.

Still another aspect of the present invention provides use of the cosmetic composition in the production of a cosmetics for improving skin wrinkles or inflammation.

The cosmetic composition, and the skin wrinkle and inflammation improvement are the same as described above.

In one embodiment of the present invention, it was confirmed that the cosmetic composition including substance P, the antioxidant, the surfactant, and the thickener of the present invention exhibited effects of promoting collagen synthesis (FIG. 3) and inhibiting collagenase (MMP-1) (FIG. 4) and effects of reducing an inflammatory cytokine IL-6 (FIG. 5) and reducing nitric oxide (NO) (FIG. 6), which are superior to those of substance P alone. Accordingly, it was confirmed to provide use of the cosmetic composition including substance P in the production of a cosmetics for improving skin wrinkles or inflammation.

### [Mode for Invention]

Hereinafter, construction and effects of the present invention will be described in more detail with reference to Examples. However, these Examples are for illustrative purposes only, and the scope of the present invention is not intended to be limited by these Examples.

### Example 1: Evaluation of stability of substance P according to contents of antioxidant and surfactant

The present inventors examined optimal contents of an antioxidant and a surfactant suitable for a cosmetic composition by performing an evaluation test of stability of substance P according to the contents of the antioxidant and the surfactant, prior to preparation of the composition for improving skin wrinkles or inflammation including substance P.

To substance P, sodium thiosulfate was added as the antioxidant by varying the content thereof (0.05%, 0.1%, 0.5%, and 1%) and polysorbate 80 was added as the surfactant by varying the content thereof (0.003%, 0.006%, 0.01%, 0.05%, and 0.1%) to prepare respective compositions, and stability of substance P in each of the formulations having the different contents of sodium thiosulfate and polysorbate 80 was measured.

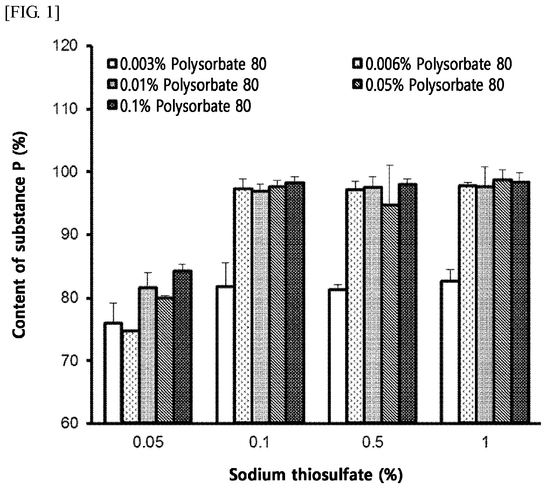

As shown in FIG. 1, it was confirmed that in the compositions including 0.1% or more of sodium thiosulfate, the stability of substance P was increased on the whole. It was also confirmed that in the compositions including 0.006% or more of polysorbate 80, the stability of substance P was increased, and the effect according to the content of polysorbate 80 was more apparent when the content of sodium thiosulfate was 0.1% or more.

### Example 2: Preparation of composition, which includes substance P, for improving skin wrinkles or inflammation

A novel formulation was prepared by adding sodium thiosulfate as the antioxidant, polysorbate 80 as the surfactant, and hydroxyethyl cellulose as the thickener to substance P. Substance P was synthesized through a solid/solution phase by Fmoc-chemistry which is a peptide synthesis technology, and purified by high performance liquid chromatography. Finally, substance P having purity of 85% or more was used.

**[Table 1]**

| Composition including substance P | |
|---|---|
| Component of composition | Content |
| Substance P | Predetermined concentration (µg/ml) |
| Sodium thiosulfate | 0.1% |
| Polysorbate 80 | 0.006% |
| Hydroxyethyl cellulose | 1.5% |

### Example 3: Examination of stability of composition including substance P in skin fibroblast culture medium

Stability of the composition including substance P was examined in a skin fibroblast culture medium. The composition including substance P (5 µg/mL) prepared according to Table 1, or substance P (5 µg/mL) dissolved in a phosphate buffer solution (PBS) as the Comparative Example was applied to a skin fibroblast culture medium (FGM, Lonza, USA) for 0 hour, 3 hours, 6 hours, 12 hours, and 24 hours, and then the content of substance P remaining in the medium was measured over time. The content of substance P was quantified by ELISA using a Substance P ELISA kit (Abcam, USA). At this time, samples applied for 0 hour were used as a control group. The control group was regarded as 100%, and the content of substance P remaining in the medium over time was calculated as a percentage (%).

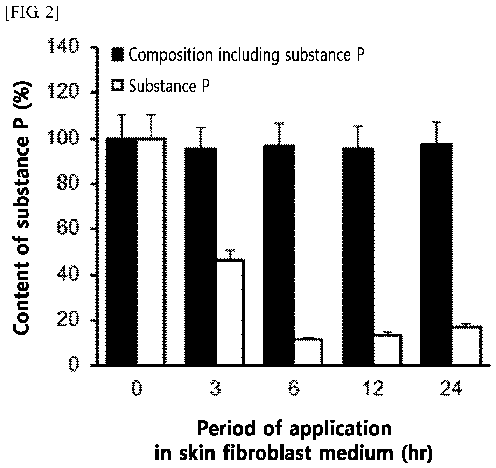

As a result, in the group treated with substance P dissolved in the phosphate buffer solution, the content of substance P was reduced up to 46% after 3-hr culture, and reduced up to 11% after 6-hr culture. In contrast, in the group treated with the composition including substance P of the present invention, the content of substance P was maintained at about 100% even after 24 hours of the culture time (FIG. 2).

These results demonstrated that the composition including substance P of the present invention may be more stable in the skin fibroblast medium, as compared to substance P alone.

### Example 4: Examination of collagen synthesis effect of composition containing substance P

The collagen synthesis effect of the composition containing substance P was examined in skin fibroblasts. Human skin fibroblasts were put in a 96-well plate at a density of 3 x 10⁴ CFU/well, and incubated at 37°C for 24 hours. The medium (FGM, Lonza, USA) was removed, and then 180 µL of a fresh medium was dispensed into each well, and 20 µL of the composition containing substance P or substance P dissolved in a phosphate buffer solution (PBS) was added thereto, followed by incubation at 37°C for 24 hours. In this regard, the concentration of the composition or the substance P added was determined such that its final concentration became a desired concentration, taking into consideration the total volume of the medium. After 24-hr culture, the supernatant was collected, and the amount of collagen in the medium was measured using a Procollagen Type1-Peptide (PIP) EIAkit (TaKaRa, Japan).

As a result, in the group treated with substance P dissolved in the phosphate buffer solution, the collagen synthesis was increased up to about 20%, after 24-hr culture. In contrast, in the group treated with the composition containing substance P of the present invention, the collagen synthesis was increased up to about 70% (FIG. 3).

The difference was statistically significant. Statistical analysis was performed using Student's t-test, and the effects of experimental groups (*p<0.05, **p<0.01) relative to the control (phosphate buffer solution-treated group) and the effects of substance P dissolved in phosphate buffer solution and the composition of the present invention (#p<0.05, ##p<0.01) were compared.

These results suggest that the composition including substance P of the present invention has more excellent collagen synthesis effect than substance P alone.

### Example 5: Examination of collagenase (MMP-1) inhibition effect of composition including substance P

The collagenase (MMP-1) inhibition effect of the composition including substance P was examined in skin fibroblasts. Human skin fibroblasts were put in a 96-well plate at a density of 3 x 10⁴ CFU/well, and incubated at 37°C for 24 hours. The medium (FGM, Lonza, USA) was removed, and then 180 µL of a fresh medium was dispensed into each well, and 20 µL of the composition including substance P or substance P dissolved in a phosphate buffer solution (PBS) was added thereto, followed by incubation at 37°C for 24 hours. In this regard, the concentration of the composition or the substance P added was determined such that its final concentration became a desired concentration, taking into consideration the total volume of the medium. After 24-hr culture, the supernatant was collected, and the amount of collagenase (MMP-1) in the medium was measured using a Human total MMP-1 kit (R&D systems, USA).

As a result, in the group treated with substance P dissolved in the phosphate buffer solution, the collagenase (MMP-1) production was decreased up to about 20%, after 24-hr culture. In contrast, in the group treated with the composition including substance P of the present invention, the collagenase (MMP-1) production was decreased up to about 40% (FIG. 4).

The difference was statistically significant. Statistical analysis was performed using Student's t-test, and the MMP-1 production of each experimental group (*p<0.05, **p<0.01) relative to the control (phosphate buffer solution-treated group) and the MMP-1 productions by substance P dissolved in phosphate buffer solution and the composition of the present invention (#p<0.05, ##p<0.01) were compared.

These results suggest that the composition including substance P of the present invention has more excellent collagenase (MMP-1) inhibition effect than substance P alone.

### Example 6: Examination of inflammatory cytokine IL-6-reducing effect of composition including substance P

The inflammatory cytokine IL-6-reducing effect of the composition including substance P was examined in Raw 264.7 cells which are mouse macrophages. Human skin fibroblasts were put in a 24-well plate at a density of 4 x 10⁵ CFU/well, and incubated at 37°C for 24 hours. A growth medium for Raw 264.7 cells was prepared by mixing DMEM (Welgene, Korea) with 200 mM of L-glutamine (Gibco, USA). After 24-hr culture, the medium was removed, and then 1 mL of a fresh medium was dispensed into each well. To induce intracellular inflammation, 100 ng/mL of LPS (Sigma, USA) was treated thereto, and simultaneously, the composition including substance P or substance P dissolved in a phosphate buffer solution (PBS) was dispensed thereto such that the final volume was 2 mL/well, followed by incubation at 37°C for 24 hours. In this regard, the concentration of the composition or the substance P added was determined such that its final concentration became a desired concentration, taking into consideration the total volume of the medium. After 24-hr culture, the supernatant was collected, and the amount of inflammatory cytokine IL-6 in the medium was measured by mouse IL-6 immunoassay (R&D systems, USA).

Significant IL-6 reduction was determined using Student's t-test by comparing the IL-6 amount practically measured in the experimental group with that of the LPS-treated group (*p<0.05, **p<0.01).

As a result, in the group treated with substance P dissolved in the phosphate buffer solution, the amount of IL-6 which was induced by the inflammation inducer LPS was decreased up to about 20%, after 24-hr culture (FIG. 5B). In contrast, in the group treated with the composition including substance P of the present invention, the amount of IL-6 which was induced by the inflammation inducer LPS was decreased up to about 50% (FIG. 5A).

These results suggest that the composition including substance P of the present invention has more excellent effect of inhibiting the inflammatory cytokine IL-6 than substance P alone.

### Example 7: Examination of nitric oxide (NO)-reducing effect of composition including substance P

In inflammatory environments, the nitric oxide (NO)-reducing effect of the composition including substance P was examined in Raw 264.7 cells which are mouse macrophages. Human skin fibroblasts were put in a 24-well plate at a density of 4 x 10⁵ CFU/well, and incubated at 37°C for 24 hours. A growth medium for Raw 264.7 cells was prepared by mixing DMEM (Welgene, Korea) with 200 mM of L-glutamine (Gibco, USA). After 24-hr culture, the medium was removed, and then 1 mL of a fresh medium was dispensed into each well. To induce intracellular inflammation, 100 ng/mL of LPS (Sigma, USA) was treated thereto, and simultaneously, the composition including substance P or substance P dissolved in a phosphate buffer solution (PBS) was dispensed thereto such that the final volume was 2 mL/well, followed by incubation at 37°C for 24 hours. In this regard, the concentration of the composition or the substance P added was determined such that its final concentration became a desired concentration, taking into consideration the total volume of the medium.

After 24-hr culture, the supernatant was collected, and 100 µL thereof was added to a 96-well plate, and an equal volume of Griess reagent (Sigma, USA) was added thereto, and the plate was left at room temperature for 15 minutes. Then, absorbance at 540 nm was measured. A calibration curve was plotted using sodium nitrite (Sigma, USA) as a standard. The production amount of nitric oxide (NO) in the group treated with LPS only was regarded as 100%, and the amount of nitric oxide (NO) in the medium was determined.

Significant NO reduction was determined using Student's t-test by comparing the NO amount practically measured in the experimental group with that of the LPS-treated group (*p<0.05, **p<0.01).

As a result, in the group treated with substance P dissolved in the phosphate buffer solution, the amount of NO which was induced by the inflammation inducer LPS was decreased up to about 10%, after 24-hr culture (FIG. 6B). In contrast, in the group treated with the composition including substance P of the present invention, the amount of NO which was induced by the inflammation inducer LPS was decreased up to about 70% (FIG. 6A).

These results suggest that the composition including substance P of the present invention has more excellent effect of inhibiting NO than substance P alone.
## Claims

1. A cosmetic composition for improving skin wrinkles or inflammation, the cosmetic composition comprising substance P comprising an amino acid sequence of SEQ ID NO: 1, an antioxidant, a surfactant, and a thickener.

2. The cosmetic composition of claim 1, wherein a concentration of the substance P is 1 µg/mL to 10 µg/mL.

3. The cosmetic composition of claim 1, wherein a concentration of the substance P is 5 µg/mL to 10 µg/mL.

4. The cosmetic composition of claim 1, wherein the antioxidant is sodium thiosulfate.

5. The cosmetic composition of claim 1, wherein a content of the antioxidant is 0.01% by weight to 1% by weight with respect to the total weight of the composition.

6. The cosmetic composition of claim 1, wherein the surfactant is polysorbate 80.

7. The cosmetic composition of claim 1, wherein a content of the surfactant is 0.001% by weight to 0.1% by weight with respect to the total weight of the composition.

8. The cosmetic composition of claim 1, wherein the thickener is hydroxyethyl cellulose.

9. The cosmetic composition of claim 1, wherein a content of the thickener is 1% by weight to 5% by weight with respect to the total weight of the composition.

10. A method of improving skin wrinkles or inflammation, the method comprising the step of applying the cosmetic composition of any one of claims 1 to 9 to the skin of a subject.

11. Use of the cosmetic composition of any one of claims 1 to 9 in the production of a cosmetic for improving skin wrinkles or inflammation.


<110> Biosolution Co.,Ltd
<120> Cosmetic composition comprising substance P for improving skin wrinkles or anti-inflammation activity
<130> OPA17111-PCT
<160> 1
<170> KoPatentIn 3.0
<210> 1
   <211> 11
   <212> PRT
   <213> Artificial Sequence
<220>
   <223> substance P
<400> 1
   Arg Pro Lys Pro Gln Gln Phe Phe Gly Leu Met
    1               5                  10